(19)

Europäisches
Patentamt
European
Patent Office
Office européen
des brevets

(11)  **EP 3 533 389 A1**

(12)  # EUROPEAN PATENT APPLICATION

(43)  Date of publication:
**04.09.2019  Bulletin 2019/36**

(21)  Application number: **18382132.1**

(22)  Date of filing: **02.03.2018**

(51)  Int Cl.:
*A61B 5/02* (2006.01)      *A61B 5/024* (2006.01)
*A61B 5/16* (2006.01)      *A61B 5/00* (2006.01)
*A61B 5/01* (2006.01)      *A61B 5/0205* (2006.01)
*A61B 5/021* (2006.01)      *A61B 5/053* (2006.01)

(84)  Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71)  Applicants:
 • **Consorcio Centro de Investigación Biomédica en Red
M.P.
28029 Madrid (ES)**
 • **Universitat Autònoma de Barcelona
08193 Bellaterra, Barcelona (ES)**
 • **Universidad De Zaragoza
50009 Zaragoza (ES)**

(72)  Inventors:
 • **Aguiló, Jordi
08193 BELLATERRA (ES)**

 • **Garzón - Rey, Jorge Mario
08193 BELLATERRA (ES)**
 • **Arza Valdés, Adriana
08830 SANT BOI DE LLOBREGA (ES)**
 • **Tsapikouni, Theodora
08193 BELLATERRA (ES)**
 • **García Pagés, Esther
08193 BELLATERRA (ES)**
 • **Kontaxis, Spyridon
50018 ZARAGOZA (ES)**
 • **Laguna Lasaosa, Pablo
50018 ZARAGOZA (ES)**
 • **Bailón Luesma, Raquel
50018 ZARAGOZA (ES)**
 • **Lázaro Plaza, Jesús
50018 ZARAGOZA (ES)**

(74)  Representative: **ZBM Patents - Zea, Barlocci & Markvardsen
Plaza Catalunya, 1 2nd floor
08002 Barcelona (ES)**

(54)  **METHODS AND SYSTEMS FOR MEASURING A STRESS INDICATOR, AND FOR DETERMINING A LEVEL OF STRESS IN AN INDIVIDUAL**

(57)  The present disclosure relates to methods, computer programs and systems for determining a stress indicator in a subject by non-invasive measurements. The stress indicator is a result from a weighted function of different parameters relating to heart rhythm variability, vasoconstriction, and sweating. In some examples, the stress indicator can indicate a level of stress on a standardized stress scale and thus provide a quick objective and reliable measurement of stress, for example acute emotional stress.

FIG. 2

EP 3 533 389 A1

**Description**

The present disclosure relates to methods, systems and computer programs for measuring a stress indicator. The present disclosure also relates to methods, systems and computer programs for measuring a level of stress in a subject. In particular, the present disclosure relates to methods and systems for measuring stress levels non-invasively and objectively. More particularly, examples of the methods and systems presented herein allow a quantitative assessment of stress levels in individuals on a scientifically validated scale.

BACKGROUND

The incidence of anxiety, depression, epilepsy, multiple sclerosis, pathological stress and many other diseases have significantly increased in recent years. Individuals all over the world are reacting physically and mentally to continuous stressful situations as a result of the modern lifestyle in a constantly changing society. The social and medical problems associated with stress are clearly growing and seriously affecting not only adults but also young and children.

Stress is a commonly-used term; however, there is no single precise definition due to the diversity of stressful stimuli and the variety of physiological reactions which should be included in the concept of stress. One of the first formal definitions, which is also the most commonly accepted definition, comes from H. Selye in 1950 "a *state of biological activation triggered by the person interacting with external agents that force his or her capacity to adapt"*. Another definition was presented by R. S. Lazarus in 1993: "*stress state arise when a specific event threatens the endocrine, physiological or psychological homeostatic stability of the individual.*"

Different types of stress exist. Firstly, the cause of stress symptoms can be different, i.e. stress can be emotional and/or physical, that is, produced by a physical or traumatic cause or on the contrary, produced by an emotion or any other psychological cause. Also, the length of time of a stress stimulus or of a stress reaction to a stressor stimulus can be different. In this sense, stress can be "acute" or "chronic". Acute stress may be defined as the immediate response to a stressor. On the other hand, stress may be regarded as chronic when the stressor or its effects on the subject are prolonged at least for a month.

Stress can have a significant effect on a person's health. Stress has been shown to increase the risk of certain somatic or psychologic disorders such as anxiety, depression, diabetes, cardiovascular risk, etc. Also, stress has been shown to change a person's general behavior and/or the response reaction to new physical or emotional stimuli and also to substantially modify the emotional state of the subject. On the other hand, it has been shown that (very) low stress levels can affect a person's wellbeing or happiness by falling to boredom, frustration, weariness, fatigue, etc. Objective and reliable measurement of the stress level can be a crucial tool for healthcare systems and for management of mental conditions for example through a continuous remote assessment for the subject follow-up.

Several methods for measuring stress have been proposed in the past. Methods and systems for evaluating stress that aim at achieving an objective assessment of stress may be divided into two different kinds of measurements. One type of evaluation is based on using psychometric tests. Psychiatry, psychology, and the medical community in general have established, and are using, such psychometric tests as the appropriate tools for assessing emotional stress. Examples of these tests include e.g. the Perceived Stress Scale (PSS) originally developed by Cohen et al., and the Depression Anxiety Stress Scales (DASS). One disadvantage of using psychometric tests, i.e. questions and answers for measuring stress, is that they take a significant amount of time and they cannot be administrated repetitively in short periods of time. The tests cannot give an indication of stress in a continuous manner. Moreover, the results of the tests are sensitive to fake answers thus making an objective assessment inviable.

Other types of stress evaluation are generally based on the measurement of biochemical markers derived from e.g. blood and saliva samples. Such biochemical markers may include e.g. catecholamine, cortisol, amylase, and some inflammation or tumor markers such as cytokines or TNF-a. An important disadvantage related to these types of measurements is that invasive methods need to be used. This can be uncomfortable for patients and because of this, in many occasions they are discarded in the current medical practice. Moreover, analyzing blood and saliva samples is time consuming and requires sophisticated laboratory equipment, so that measurements can only be done involving e.g. healthcare facilities. In summary, this kind of information source can't be used for continuous assessment in a normal life environment.

There are further methods known that rely on non-invasive measurements such as e.g. heart rate, transpiration or pupil dilatation as rough indicators of stress. To measure transpiration, it is known to measure e.g. skin conductance. However, such a measurement cannot give a reliable, repetitive and objective measurement of stress because it is influenced by many other external uncontrollable factors.

Moreover, there is no generally accepted stress reference scale that can objectively indicate, the level of stress suffered by an individual and allowing a quantitative comparison with other individuals. In most studies, the determination of stress does not go beyond a determination of "stress" or "no stress".

Therefore, there is a need for systems and methods for determining stress that at least partially reduce some of the aforementioned drawbacks.

SUMMARY

In a first aspect, the present disclosure provides a method of measuring a stress indicator in a subject comprising non-invasively and synchronously measuring one or more heart rhythm parameters indicative of a heart rhythm variability of the subject, one or more sweat parameters indicating a level of perspiration of the subject, and one or more arterial parameters indicating a level of widening or narrowing of blood vessels in the subject. The method further comprises determining a weighted function of the heart rhythm variability parameters, the sweat parameters and the arterial parameters to define the stress indicator.

In accordance with this aspect, the combination of different parameters can give accurate information on changes in the hypothalamic-pituitary-adrenal axis (HPA axis) and on the autonomic nervous system (ANS) of the subject and thus give a reliable indication of stress. In accordance with this aspect, user-friendly methods (since they are non-invasive) for measuring a stress indicator are provided. Moreover, the stress measurement can be carried out within a short time frame, even continuously if needed. A quantitative assessment of stress remotely is also made possible.

A reliable and user-friendly stress measurement can be useful for health management, e.g. stress can be measured rather than having to rely on a patient's impression and can also facilitate an easy and precise communication among professionals in order to find more suitable and personalized solutions for the treatment of pathological cases. Such a stress measurement may further be useful to test e.g. effectiveness of a drug for reducing stress.

In accordance with this aspect, the measurements are synchronized: the measurements are made at the same time and the signals received from different sensors are synchronized with each other so that measurements from one sensor may be correlated in time with measurements from another sensor.

Herein, heart rhythm variability is to be understood as a variation in the time interval between heartbeats.

In some examples, the heart rhythm parameters also include parameters indicative of an average heart rhythm. A mean heart rhythm over a measuring period is a further parameter that can indicate stress.

In some examples, the weighted function of mean heart rhythm, heart rhythm variation parameters, the sweat parameters and the arterial parameters is a weighted function of normalized parameters. Normalization of the parameters may serve to adjust the values measured on different scales to a common scale. Normalization of the parameters may be done in accordance with the following Equation:

$$x^{\wedge}_{i} = \frac{x_i - X_{min}}{(X_{max} - X_{min})} \cdot 100 \qquad\qquad (Eq.\ 1)$$

Wherein

$x_i$: Value of the variable x concerning participant i on the set X

$[\![\,x^{\wedge}\,]\!]_i$: $x_i$ rescaled value

$X_{min}$, $X_{max}$: Minimum and maximum value of the set X

A weighted function may be a weighted sum or a weighted product of parameters, and may also be a combination of a weighted sum and a weighted product. The weighted function may comprise linear and non-linear combinations of parameters.

In some examples, the weighted function indicates a level of stress on a standardized stress scale, optionally the perceived stress scale (PSS), the Visual Analogue Scale for Stress (VASS), or the State-Trait Anxiety Inventory test (STAI), or a combination of standardized stress scales. Different standardized stress scales exist, which usually rely on psychometric tests.

"Calibration" of the stress indicator may take place by correlating test results from psychometric stress tests and/or biochemical markers with stress test results relating to the non-invasive parameters. A statistical data analysis based on a machine learning process involving e.g. factor analysis may be used to determine the weighted sum of parameters which defines the stress indicator can then give a value on a known stress scale.

In examples, a stress scale may be defined by combining different standardized stress tests, and optionally further combining with known biochemical markers for stress, such as e.g. Copeptin, prolactin and glucose and cortisol and $\alpha$-amylase (obtainable from a blood or saliva sample). For example, one such a stress scale may be defined by the following equation:

$$Stress_{ref} = \frac{1}{5}(\Delta Cortisol\char`\^ + Prolactin\char`\^ + Copeptin\char`\^ + VASS\char`\^ + STAIs\char`\^) \qquad (Eq.\ 2)$$

Other such stress scales may be defined by a weighted function, in particular a weighted sum, optionally with different weight factors of known stress scales, and one or more biochemical markers related to stress. Such stress scales, as defined above, may be defined on normalized values of the stress scales and biochemical markers.

Depending on the precision required for a given stress measurement application, calculation and calibration may be only very roughly done or very precisely done. That is, according to the requirements, a different weighted average may be defined for different groups of subjects. For example, for follow-up purposes calibration or reference could be done through past obtained values while for independent measures values obtained would be referred to known standard values for this kind of population.

These groups may be defined by e.g. sex, age, race and other factors.

In some examples, the weighted function of the sweat parameters, heart rhythm parameters and arterial parameters is determined in a process of machine learning of data from standardized stress tests, optionally the Trier Social Stress Test (TSST), wherein the data includes data on biochemical markers and psychometric tests of a plurality of subjects in the standardized stress test. Machine learning and/or data mining may be used to determine the weighted function such that the weighted function of a given set of parameters can be linked to stress levels as determined in psychometric tests and/or levels of biochemical markers in standardized stress tests.

In some examples, the one or more heart rhythm parameters may include one or more of the following:

- a parameter indicative of the time variation (e.g. a standard deviation) in the heart rate, derived e.g. from the ECG or a direct heart rate recording during a measuring period;

- a parameter indicative of the time variation (e.g. a standard deviation) in the pulse period derived from a PPG signal during a measuring period;

- a parameter indicative of a power spectral density (PSD) of the heart rate variability, in particular spectral power in a very low frequency band, e.g. from 0.003 Hz to 0.04 Hz and spectral power in a low frequency band, e.g. from 0.04 Hz to 0.15 Hz;

- a parameter indicative of a pulse transit time (PTT) or indicative of a variability in the pulse transit time, the pulse transit time, PTT, indicates the time distance between an R peak in an ECG signal, and a reference point between a basal and apex amplitude in a PPG signal following the R peak.

In some examples, additional information on the arterial or circulatory system may be derived from one or more arterial parameters selected from following:

- a parameter relating to a skin temperature at a first location in a body of the subject that is prone to vasoconstriction,

- a parameter indicative of a comparison between a skin temperature at the first location prone to vasoconstriction in the body of the subject and a skin temperature at a second location prone to vasodilation in the body of the subject,

- a parameter indicative of the pulse transit time (PTT) or indicative of a variation in the pulse transit time, the pulse transit time indicating a length of time between a peak in the R signal in the electrocardiogram, and some predefined reference point in the corresponding pulse of photoplethysmography (PPG) signal following the peak; for example, the midpoint between the basal and apex amplitude

- a parameter indicative of a variation in the pulse rising time (PRT), the pulse rising time indicating a length of time between a basal point and an apex point in a PPG signal, and

- a parameter indicative of a variation in a pulse width until reflection (PWR), the pulse width until reflection indicating a length of time between a basal point and a dichrotic notch point in a PPG signal.

- a parameter indicative of an average pulse wave decreasing time in a PPG signal, the pulse wave decreasing time corresponding to a length of time between a peak and a subsequent basal point in a PPG signal.

In one particular example, the combination of heart rhythm variability parameters, the sweat parameters and the arterial parameters include:

- a parameter indicative of a comparison between a skin temperature at the first location prone to vasoconstriction in the body of the subject and a skin temperature at a second location prone to vasodilation in the body of the subject,

- a parameter indicative of a variation in the pulse rising time (PRT), the pulse rising time indicating a length of time between a basal point and an apex point in a pulse photoplethysmography (PPG) signal,

- a parameter indicative of the pulse transit time (PTT) and a parameter indicative of a variation in the pulse transit time, the pulse transit time indicating a length of time between a peak in the R signal in an electrocardiogram, and a mid point between a basal and apex amplitude in a pulse photoplethysmography (PPG) signal following the peak,

- a parameter indicative of a variation (e.g. the standard deviation) in a pulse width until reflection (PWR), the pulse width until reflection indicating a length of time between a basal point and a dichrotic notch point in a pulse photoplethysmography (PPG) signal; and

- a parameter indicating skin conductance.

Inventors have found that this combination of parameters can give a particularly reliable measurement of stress.

In some examples, the parameter indicative of a comparison between a skin temperature at the first location prone to vasoconstriction in the body of the subject and a skin temperature at a second location prone to vasodilation in the body of the subject is a ratio between a skin temperature of a finger of a subject and a skin temperature of a cheek of the subject. The inventors have found that this ratio is a parameter which is a good indicator of stress, i.e. the ratio stays constant in the absence of stress and start to change at the occurrence of stress. Another parameter that has proven to be a good indicator is a parameter indicating temperature variation in a location prone to vasoconstriction.

In a further aspect, the present disclosure provides a method, in particular a computer-implemented method, for diagnosing stress comprising measuring a stress indicator in accordance with any of the examples described herein, comparing the measured stress indicator with a predefined threshold for the stress indicator; and diagnosing stress if the measured stress indicator is above the predefined threshold. Such a method may be completely automated. In such a case, one or more predefined thresholds might be stored in a computer memory.

In yet a further aspect, a computer program for determining a stress indicator in a subject is provided. The computer program comprising instructions which, when the program is executed by a computer, cause the computer to carry out a method comprising: receiving from one or more sensors heart rhythm signals indicative of a heart rhythm of the subject; receiving from one or more sensors perspiration signals indicative of a level of perspiration of the subject; and receiving from one or more sensors blood vessel signals indicative of a level of widening or narrowing of blood vessels in the subject. The method further comprises determining a stress indicator in accordance with any of the examples disclosed herein. In particular, the method further comprises determining from the heart rhythm signals one or more heart rhythm parameters indicative of heart rhythm variability, determining from the perspiration signals one or more sweat parameters, determining from the blood vessel signals one or more arterial parameters, indicative of a level of widening or narrowing of blood vessels in the subject, and determining a weighted function of the heart rhythm parameters, the sweat parameters and the arterial parameters to define the stress indicator.

In yet a further aspect, the present disclosure provides a system for determining a stress indicator in a subject. The system comprises one or more heart rhythm sensors for non-invasively measuring a heart rhythm of the subject, one or more sweat sensors for non-invasively measuring a level of perspiration of the subject, and one or more arterial sensors for non-invasively measuring one or more arterial parameters indicating a level of widening or narrowing of blood vessels in the subject. The system furthermore comprises a computing module which is configured to receive heart rhythm signals indicative of a heart rhythm variability of the subject from the heart rhythm sensors, perspiration signals indicative of a level of perspiration of the subject from the sweat sensors, and signals indicative of a level of widening or narrowing of blood vessels in the subject from the arterial sensors blood vessel. The computing module is furthermore configured to determine a stress indicator in accordance with any of the methods herein described.

In accordance with this aspect, a device or system able to measure a set of electrophysiological signals needed to calculate the right biomedical features concerning stress measurement is provided. The device may comprise specific sensors, electronics for signal acquisition and conditioning, electronic modules for extraction of the right features from signals, memory devices to save registered data, and a computing module to calculate the stress level of the individual as well as to generate appropriate sets of data to be displayed.

In some examples, the computing module may further store in the memory records about stressor types, sociodemographic data and history of evolution (if existing) from individuals being monitored or tested.

In some examples, the devices or systems may include data transmission capabilities to send an indicator of a stress level to a user's mobile phone or Smartphone, tablet or personal computer or any other device having data transmission capabilities, to a healthcare facility (e.g. a doctor's office or a hospital) or to a website dedicated to tracking of stress levels in individuals or groups of people.

In some examples, the heart rhythm sensors include one or more of the following: an ECG sensor, a PPG sensor, an elastic band for respiratory rate measurement, a digital camera for capturing an image from the subject, and a microphone recording heart beats from the subject, and/or recording a voice from the subject. Data regarding average heart rhythm during a measuring period after exposure to a stressor and data regarding heart rhythm variations may be directly derived from an ECG. Another sensor that can register a heart rhythm directly is a microphone which might be incorporated in e.g. a (digital) stethoscope. However, other sensors can also be used because they provide an indirect measurement of a heart rhythm (e.g. a pulse oximeter) or measure parameters that are affected by heart rhythm (variations), such as e.g. a respiratory rate sensor. Yet a further possibility is for a digital image of a subject to be recorded. Through suitable image processing, e.g. respiratory rate may be measured, in such a way that heart rate can be extracted from respiratory flow signal. Yet a further possibility is to record a voice of a subject while talking. Digital processing can reveal heart rhythm changes from changes in voice pattern. Also, various of these sensors may be combined. For example, a combination of measuring a respiratory rate and an ECG can make the heart rate measurement more reliable.

In some examples, the sweat sensors include one or more of the following: a skin conductance sensor, an impedance sensor, a digital camera or a thermal imaging camera. Different types of sensors could be used to indicate a level of perspiration in a subject. Electrodermal activity may be measured directly by measuring skin conductance (increased perspiration will lead to a lower electrical resistance between two points on the skin) or e.g. by measuring impedance. Another parameter that may be used may be e.g. a number of sweat droplets in a specific skin area as determined by image processing of a camera,

In some examples, the arterial sensors may include one or more of the following: pulse oximeter, thermometer, a thermal imaging camera, an ultrasound transducer, or a microflow imaging device.

In a particular example, the sensors comprise (only) a PPG sensor, an ECG sensor, one or more thermometers for measuring skin temperature and a skin conductance sensor. It has been found that a combination of these few sensors, which are off-the-shelf, reliable, and affordable can give a very reliable measurement of stress. Heart rhythm parameters can be derived from the ECG and to some extent from the PPG. Sweat parameters can be derived from the skin conductance sensor and parameters relating to vasoconstriction or vasodilation can be derived from a PPG, and from skin temperature measurements, and particularly from skin temperature measurement in a location where vasoconstriction could take place, e.g. a finger.

BRIEF DESCRIPTION OF THE DRAWINGS

Non-limiting examples of the present disclosure will be described in the following, with reference to the appended drawings, in which:

Figure 1 schematically indicates a plurality of parameters that may be extracted from a PPG and an ECG signal that can be used in examples of methods and systems for measuring a stress indicator;

Figure 2 schematically indicates an example of a system for determining a stress indicator;

Figure 3 schematically indicates examples of different sensors that may be used in examples of systems for determining a stress indicator; and

Figure 4 schematically indicates an example of a wearable device incorporating a system for determining a stress indicator.

DETAILED DESCRIPTION OF EXAMPLES

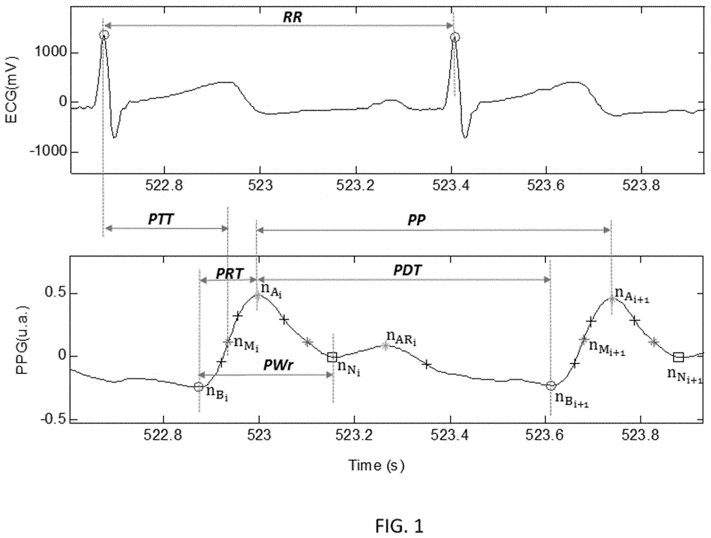

Figure 1 schematically indicates a plurality of parameters that may be extracted from a PPG and an ECG signal that can be used in examples of methods and systems for measuring a stress indicator.

A number of characteristic points may be defined for the ith pulse in the PPG and ECG signals:

$n_{A_i}$ : Apex point defined as the maximum of the pulse. Each absolute maximum of the PPG signal between two successive QRS complexes detected in the ECG signal is considered to be an apex point of a PPG pulse.

$n_{B_i}$: Basal point, defined as the absolute minimum of the PPG signal between 2 successive QRS complexes in the ECG signal.

$n_{M_i}$: Middle point, defined as the instant in time when the PPG pulse reaches half of its apex-to-basal amplitude.

$n_{AR_i}$: Apex point of the reflected wave, defined as the maximum reflected pulse wave.

$n_{N_i}$: Dichrotic notch point, defined as the inflection point between pulse wave and reflected wave.

Having defined these points, the following parameters can be defined:

1) Heart Rate: inverse of time interval RR
2) Pulse Period (PPi): time interval between two consecutive $n_{A_i}$. Both the heart rate and pulse period may alternatively be measured by choosing different points in the ECG or PPG signal.
3) Pulse Rate (PRi): inverse of PPi
4) Pulse Transit Time ($PTT_i$): time interval between the maximum of the R wave in the ECG signal and its associated $n_{M_i}$. PTT is inversely proportional to pulse wave velocity, which is associated with arterial stiffness and cardiovascular output, and therefore a decrease in PTT is related to an increase in blood vessel resistance and cardiovascular output, as well as being inversely related to blood pressure. Pulse Transit Time is indicative of the balance of sympathetic and parasympathetic systems and of the instantaneous circulatory system performance.
5) Pulse Wave Rising Time ($PRT_i$): time interval between $n_{B_i}$ and $n_{A_i}$. PRT is an index of the pulse wave and is related to arterial stiffness, blood volume and systolic blood pressure.
6) Pulse Width until Reflected Wave (PWn): time interval between $n_{B_i}$ and $n_{N_i}$. PWR is a time index of the reflected pulse wave arrival and is associated with PRT, and also with peripheral central vascular resistance and the cardiovascular output, as well as pulse wave velocity.
7) Pulse Wave Decreasing Time ($PDT_i$): defined as the time interval between $n_{A_i}$ and $n_{B_{i+1}}$. PDT is an index of diastolic time, also highly related to Pulse Rate

Apart from these parameters, parameters indicative of variability in the parameters may be defined, e.g. standard deviation and root mean square of the successive differences. Also, frequency parameters may be used, e.g. LF power (PLF) and HF power (PHF), in time windows of e.g. 1 minute, from the power spectral density (PSD) of the heart rate variability signal (HRV). The PSD was calculated using Fourier transformation.

The HRV signal is obtained from the instantaneous HR signal, derived from the beat occurrence time series using the integral pulse frequency modulation model, subtracting from the instantaneous HR signal, a low pass filtered HR signal (cut-off frequency of 0.03Hz), which mainly accounts for changes in mean HR.

The LF/HF ratio was also computed. Power in the very low frequency (PVLF) band, ranging from 0 to 0.03Hz, was computed from the power spectral density of the low pass filtered HR signal.

ECG and PPG signals may further be combined with skin temperature measurements: temperature measurements may be carried out in particular in locations which normally show vasoconstriction, such as e.g. a finger. Further temperature measurements may be carried out in a location more prone to vasodilation e.g. in the cheek of a subject.

Suitable parameters include:

1) $T_{fa}$ Face temperature,
2) $T_{fi}$ Finger temperature
3) Temperature Gradient, ($\Delta T$): Temperature variations during every period, e.g. 1 minute, obtained as:

$$\Delta T_i = T_{i+1} - T_i$$

Where: $T_i$ represent temperature values every 1 minute, obtained as the mean value in a time window of 1 second in the minute i
4) Temperature power ($T_P$) : defined as the average power in a time window of e.g. 1 minute.
5) Ratio of finger and face temperature ($T_{ratio}$): defined as mean value in a time window of e.g. 1 minute.

$$T_{ratio_i} = T_{fi_i} / T_{fa_i}$$

Within the framework of the present development, in one particular validation experiment, these parameters were measured in a plurality of individuals undergoing standardized stress test, namely a version of the Trier Social Stress Test (TSST). Apart from measuring these parameters, also biochemical markers known to be indicative of stress were measured and the individuals underwent psychometric tests as well. By correlating the measured parameters, the following weighted average was identified as a suitable stress indicator, SI, such as:

$$SI = 0.06\ PTT_i + 0.4\ \Delta T_{fi} + 0.4\ Heart\ Rate + 0.2\ PHF \qquad (Eq.\ 3)$$

This combination of parameters fulfils the following preferred criteria:

- The measured parameter has a strong correlation value measured stress levels after or during exposure to a stressor
- The measured parameter does not show strong variations when there is no exposure to a stressor
- There is no linear interdependency between them

It should be clear however, that other combinations of parameters are also possible, with different weighing factors. In particular, linear dependency was found between the following parameters:

$$(1)\ T_{fi_{Ptotal}};\ T_{Ratio}$$

$$(2)\ HR_{mean};\ HR_{rMSSD};\ HR_{PVLF}\ ;\ PR_{mean};\ PR_{rMSSD};\ PR_{PVLF};\ PDT_{mean}.$$

In another validation experiment related to chronic stress, individuals of the taken sample were continuously exposed to some stressor stimuli for more than two months. Biochemical markers known to be indicative of stress were measured and the individuals underwent psychometric tests as well. Additionally, the same signals mentioned in the previously explained experimental pilot were registered and related parameters calculated. That is, Heart Rate, Pulse Period (PPi), Pulse Rate (PRi), Pulse Transit Time ($PTT_i$), and all the others including those related to the variability such as LF power (PLF) and HF power (PHF), LF/HF ratio HR and all others extracted from ECG and PPG. In the same way parameters related to skin temperature as well as those related to vasoconstriction and vasodilation were also measured.

The range of values calculated from an appropriate combination of these parameters with slightly different weighing factors than in the previous experiment match with the stress level found on a standardized stress scale.

It was found that for different types of stress, i.e. physical vs emotional and chronic vs acute, the same or similar parameters were useful. A similar parameter should herein be understood as a parameter giving similar information. For the different types of stress, slightly different weighing factors were determined.

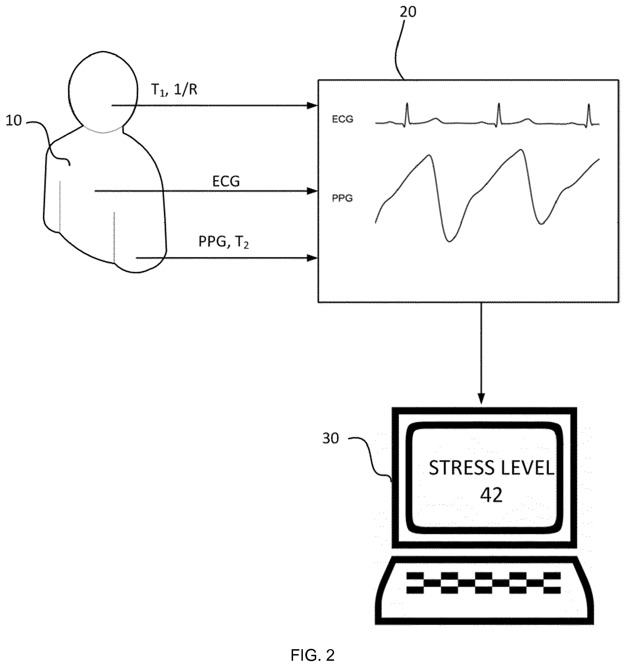

There are different possible implementations for a stress indicator determined in accordance with the principles described herein. Figure 2 schematically indicates an example of a system for determining a stress indicator. A plurality of sensors may be attached to subject 10. In particular, in this example, skin temperature may be measured in the face and finger of the subject. At the same time, an ECG and PPG were measured. Also skin conductance is measured.

Signals representing the above measurements may be sent to computing module 20. Computing module 20 may be configured to receive the signals, and derive appropriate parameters relating to perspiration, heart rate variation, vasoconstriction / vasodilation. The computing module 20 may further be configured to calculate the stress indicator and sent a signal to a display 30 capable of reproducing the signal.

The display may be a computer screen, a television screen, a tablet, a mobile phone, Smartphone or similar.

In some examples, the computing module may be configured to give a stress diagnosis, e.g. by comparing a calculated stress level to one or more predetermined thresholds. The display may reproduce a stress level in the form of a number or a qualitative scale, e.g. "any stress", "stress", "high level of stress" "acceptable level of stress".

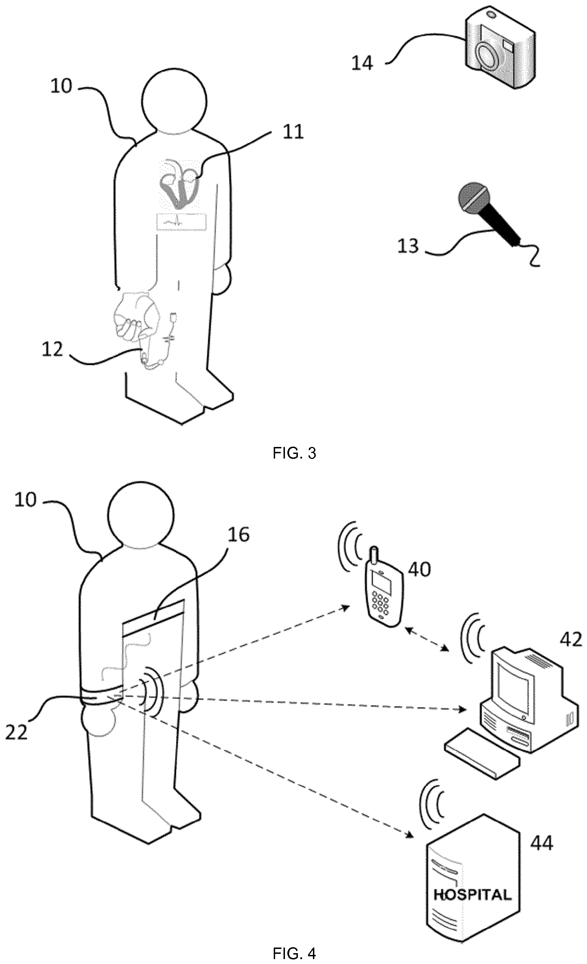

Instead of the measurements schematically indicated in figure 2, other sensors could also be used. For example, Figure 3 schematically indicates examples of different sensors that may be used in examples of systems for determining a stress indicator. Subject 10 carries an ECG sensor 11 (just as in figure 2) and a pulse oximeter 12 (also same as in figure 2). Alternatively, or in addition, a digital camera or thermal imaging camera 13 can be used. Further alternatively, or additionally, a microphone 14 can be used. Signals may be sent to a computing module such as the one shown in figure 2.

The computing module in this system may be configured to process the received signals and determine e.g.:

- Heart rate from microphone. The microphone could directly record a heart beat, or a heart rate may be derived from voice analysis when the subject is talking.
- Skin temperature in various locations from a thermal imaging camera

- Data on perspiration from image processing from a digital camera. Image processing might also be used to derive e.g. respiratory rate.

Systems similar to the examples of figures 2 and 3 might be used e.g. in a general practitioner's office. Another implementation includes a regular check of stress levels of e.g. airline pilots, or bus drivers. From a suitable combination of sensors, the relevant parameters may be extracted. An objective indication can be given of an individual's stress level. Both very high or very low levels might be a reason to impede an airline pilot to fly or a bus driver to drive. Similar tests could be envisioned for any professional who is a position of high responsibility.

Figure 4 schematically indicates an example of a wearable device 22 incorporating a system for determining a stress indicator. The wearable device may take the form of a bracelet or watch. Such a device 22 might be equipped to e.g. measure a PPG signal. Further functionalities that could be included are e.g. a thermometer to measure skin temperature and a conductance or impedance sensor. Additional data could be received from an elastic band 16 measuring a respiratory rate. Such an elastic band may have a wireless connection with wearable device 22. The wearable device may further comprise suitable software and a processor for determining the different parameters and deriving a weighted average of chosen parameters.

The wearable device may further include a memory in which different weighted sums for the stress indicator may be stored. A user might input information on age, sex etc. so that the wearable can calculate the applicable weighted sum.

The calculated stress indicator might be displayed on wearable device 22. And data from the measurements and/or information related to stress level might be sent to a mobile phone or Smartphone 42, to a computer system 44 (or a dedicated website) and/or e.g. to a server from a healthcare facility. Both an individual consumer and e.g. a doctor can thus continuously obtain information on a stress level.

For completeness, various aspects of the present disclosure are set out in the following numbered clauses:

Clause 1. A method of measuring a stress indicator in a subject comprising non-invasively and synchronously measuring
one or more heart rhythm parameters indicative of a heart rhythm variability of the subject,
one or more sweat parameters indicating a level of perspiration of the subject, and
one or more arterial parameters indicating a level of widening or narrowing of blood vessels in the subject; and
determining a weighted function of the heart rhythm variability parameters, the sweat parameters and the arterial parameters to define the stress indicator.

Clause 2. The method according to clause 1, wherein the heart rhythm parameters also include parameters indicative of an average heart rhythm.

Clause 3. The method according to clause 1 or 2, wherein the weighted function of heart rhythm parameters, the sweat parameters and the arterial parameters is a weighted function of normalized parameters, optionally a weighted sum or weighted product, or a combination of these comprising linear and non-linear combinations

Clause 4. The method according to any of clauses 1 - 3, wherein the weighted function indicates a level of stress on a stress scale, optionally a standardized stress scale, such as the perceived stress scale (PSS), the Visual Analogue Scale for Stress (VASS), or the State-Trait Anxiety Inventory test (STAI) or a combination of standardized stress scales.

Clause 5. The method according to any of clauses 1 - 3, wherein the weighted function indicates a level of stress on a combined stress scale, wherein the combined stress scale is a weighted function of one or more standardized stress scales and biochemical markers related to stress.

Clause 6. The method according to clause 4 or 5, wherein the weighted function is determined in a process of machine learning of data from stress tests, particularly standardized stress tests, optionally the Trier Social Stress Test (TSST), wherein
the data includes data on biochemical markers and psychometric tests of a plurality of subjects in the standardized stress test.

Clause 7. The method according to any of clauses 4 - 6, further comprising displaying the level of stress.

Clause 8. The method according to any of clauses 1 - 7, wherein the stress indicator is used to indicate acute emotional stress.

Clause 9. A method for diagnosing stress comprising
measuring a stress indicator in accordance with any of clauses 1 - 8,
comparing the measured stress indicator with a predefined threshold for the stress indicator; and
diagnosing stress if the measured stress indicator is above the predefined threshold.

Clause 10. The method according to any of clauses 1 - 9, wherein the one or more sweat parameters include one or more of the following: skin impedance, skin conductance, dimensions or number of sweat droplets derived from a digital image.

Clause 11. The method according to any of clauses 1 - 10, wherein the one or more heart rhythm parameters include one or more of the following:

- a parameter indicative of the time variation in the heart rate during a measuring period;
- a parameter indicative of the time variation in the pulse period derived from a PPG signal during a measuring period;
- a parameter indicative of a power spectral density (PSD) of the heart rate variability, in particular a power in a low or very low spectral band;
- a parameter indicative of a pulse transit time (PTT) or indicative of a variability in the pulse transit time, the pulse transit time indicating a length of time between an R peak in an ECG signal, and a reference point between a basal and apex amplitude in a pulse photoplethysmography (PPG) signal following the R peak.

Clause 12. The method according to any of clauses 1 - 11, wherein the one or more arterial parameters include one or more of the following:

- a parameter relating to a skin temperature at a first location in a body of the subject that is prone to vasoconstriction,
- a parameter indicative of a comparison between a skin temperature at the first location prone to vasoconstriction in the body of the subject and a skin temperature at a second location prone to vasodilation in the body of the subject,
- a parameter indicative of an average pulse transit time (PTT) or indicative of a variation in the pulse transit time, the pulse transit time indicating a length of time between a peak in the R signal in an electrocardiogram, and a mid point between a basal and apex amplitude in a pulse photoplethysmography (PPG) signal following the peak,
- a parameter indicative of a variation in the pulse rising time (PRT), the pulse rising time indicating a length of time between a basal point and an apex point in a pulse photoplethysmography (PPG) signal,
- a parameter indicative of an average pulse wave decreasing time in a PPG signal, the pulse wave decreasing time corresponding to a length of time between a peak and a subsequent basal point in a PPG signal; and
- a parameter indicative of a variation in a pulse width until reflection (PWR), the pulse width until reflection indicating a length of time between a basal point and a dichrotic notch point in a pulse photoplethysmography (PPG) signal.

Clause 13. The method according to any of clauses 1 - 12, wherein the sweat parameters and the arterial parameters include:

- a parameter indicative of a skin temperature in the first location prone to vasoconstriction,
- a parameter indicative of a pulse transit time (PTT), the pulse transit time indicating a length of time between a peak in the R signal in an electrocardiogram, and a reference point between a basal and apex amplitude in a pulse photoplethysmography (PPG) signal following the peak
- a parameter indicating an average heart rhythm.

Clause 14. The method according to clause 13, wherein the parameter indicative of a skin temperature in the first location prone to vasoconstriction is one of the following:

- a parameter indicative of a comparison between a skin temperature at the first location prone to vasoconstriction in the body of the subject and a skin temperature at a second location prone to vasodilation in the body of the subject,
- a parameter indicative of a variation of a skin temperature in the first location prone to vasoconstriction, particularly a finger of the subject.

Clause 15. The method according to any of clauses 1 - 12, wherein the heart rhythm variation parameters, the sweat parameters and the arterial parameters include:

- a parameter indicative of a comparison between a skin temperature at the first location prone to vasoconstriction in the body of the subject and a skin temperature at a second location prone to vasodilation in the body of the subject,
- a parameter indicative of a variation in the pulse rising time (PRT), the pulse rising time indicating a length of time between a basal point and an apex point in a pulse photoplethysmography (PPG) signal,
- a parameter indicative of an average pulse transit time (PTT) and a parameter indicative of a variation in the pulse transit time, the pulse transit time indicating a length of time between a peak in the R signal in an electrocardiogram, and a reference point between a basal and apex amplitude in a pulse photoplethysmography (PPG) signal following the peak,
- a parameter indicative of a variation in a pulse width until reflection (PWR), the pulse width until reflection indicating a length of time between a basal point and a dichrotic notch point in a pulse photoplethysmography (PPG) signal
- a parameter indicating skin conductance.

Clause 16. The method according to any of clauses 12 - 15, wherein the parameter indicative of a variation in the pulse transit time, is a standard deviation of the pulse transit time during the measuring period.

Clause 17. The method according to any of clauses 12 - 16, wherein the parameter indicative of a variation in the pulse rising time (PRT) is a standard deviation of the pulse rising time during the measuring period.

Clause 18. The method according to any of clauses 12 - 17, wherein the parameter indicative of a variation in a pulse width until reflection (PWR), is a standard deviation of the pulse width until reflection.

Clause 19. A computer program for determining a stress indicator in a subject, the computer program comprising instructions which, when the program is executed by a computer, cause the computer to carry out a method comprising:

- receiving from one or more sensors heart rhythm signals indicative of a heart rhythm of the subject;
- receiving from one or more sensors perspiration signals indicative of a level of perspiration of the subject;
- receiving from one or more sensors blood vessel signals indicative of a level of widening or narrowing of blood vessels in the subject;
- determining from the heart rhythm signals one or more heart rhythm parameters indicative of heart rhythm variability,
- determining from the perspiration signals one or more sweat parameters,
- determining from the blood vessel signals one or more arterial parameters, indicative of a level of widening or narrowing of blood vessels in the subject,
- determining a weighted function of the heart rhythm parameters, the sweat parameters and the arterial parameters to define the stress indicator.

Clause 20. The computer program according to clause 19, wherein the weighted function of the heart rhythm parameters, the sweat parameters and the arterial parameters is a weighted combination of normalized parameters, optionally comprising linear and non-linear combinations.

Clause 21. The computer program according to clause 19 or 20, wherein the weighted function indicates a level of stress on a standardized stress scale, optionally the perceived stress scale (PSS), the Visual Analogue Scale for Stress (VASS), or the State-Trait Anxiety Inventory test (STAI), or a combination of standardized stress scales.

Clause 22. The computer program according to any of clauses 19 - 21, wherein the weighted function of parameters indicates a level of stress on a combined stress scale, the combined stress scale being a weighted sum of standardized stress scales and biochemical markers related to stress.

Clause 23. The computer program according to any of clauses 19 - 22, wherein the weighted sum is determined in a process of machine learning of data from (standardized) stress tests, optionally the Trier Social Stress Test (TSST), wherein the data includes data on biochemical markers and psychometric tests of a plurality of subjects in the standardized stress test.

Clause 24. A system for determining a stress indicator in a subject comprising

- one or more heart rhythm sensors for non-invasively measuring a heart rhythm of the subject;
- one or more sweat sensors for non-invasively measuring a level of perspiration of the subject;
- one or more arterial sensors for non-invasively measuring one or more arterial parameters indicating a level of widening or narrowing of blood vessels in the subject; and
- a computing module for

  - receiving from one or more sensors heart rhythm signals indicative of a heart rhythm of the subject;
  - receiving from one or more sensors perspiration signals indicative of a level of perspiration of the subject;
  - receiving from one or more sensors blood vessel signals indicative of a level of widening or narrowing of blood vessels in the subject;
  - determining from the heart rhythm signals one or more heart rhythm parameters indicative of heart rhythm variability,
  - determining from the perspiration signals one or more sweat parameters,
  - determining from the blood vessel signals one or more arterial parameters, indicative of a level of widening or narrowing of blood vessels in the subject,
  - determining a weighted function of the heart rhythm parameters, the sweat parameters and the arterial parameters to define the stress indicator.

Clause 25. The system according to clause 24, wherein the heart rhythm sensors include one or more of the following: an ECG sensor, a PPG sensor, an elastic band for respiratory rate measurement, a digital camera for capturing an image from the subject, and a microphone recording heart beats from the subject, or recording a voice from the subject.

Clause 26. The system according to clause 24 or 25, wherein the sweat sensors include one or more of the following: a skin conductance sensor, an impedance sensor, a digital camera or a thermal imaging camera.

Clause 27. The system according to any of clauses 24 - 26, wherein the arterial sensors include one or more of the following: a PPG sensor, thermometer, a thermal imaging camera, an ultrasound transducer, or a microflow imaging device.

Clause 28. The system according to clause 24, wherein the sensors comprise a pulse oximeter, an ECG sensor, and one or more thermometers for measuring skin temperature and optionally a skin conductance sensor.

Clause 29. The system according to any of clauses 24 - 28, wherein the system is integrated in a wearable device.

Clause 30. The system according to any of clauses 24 - 29, further comprising a display.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

**Claims**

1. A method of measuring a stress indicator in a subject comprising non-invasively and synchronously measuring one or more heart rhythm parameters indicative of a heart rhythm variability of the subject, one or more sweat parameters indicating a level of perspiration of the subject, and one or more arterial parameters indicating a level of widening or narrowing of blood vessels in the subject; and determining a weighted function of the heart rhythm variability parameters, the sweat parameters and the arterial parameters to define the stress indicator.

2. The method according to claim 1, wherein the heart rhythm parameters also include one or more parameters indicative of an average heart rhythm.

3. The method according to claim 1 or 2, wherein the weighted function of heart rhythm parameters, the sweat parameters and the arterial parameters is a weighted function of normalized parameters, optionally a weighted sum of the normalized parameters. comprising linear and non-linear combinations.

4. The method according to any of claims 1 - 3, wherein the weighted sum indicates a level of stress on a combined stress scale, wherein the combined stress scale is a weighted sum of one or more standardized stress scales and one or more biochemical markers related to stress.

5. The method according to claim 3 or 4, wherein the weighted sum is determined in a process of machine learning of data from stress tests, , wherein
the data includes data on biochemical markers and psychometric tests of a plurality of subjects in the standardized stress test.

6. A computer implemented method for diagnosing stress comprising
measuring a stress indicator in accordance with any of claims 1 - 5,
comparing the measured stress indicator with a predefined threshold for the stress indicator; and
diagnosing stress if the measured stress indicator is above the predefined threshold.

7. The method according to any of claims 1 - 6, wherein the one or more heart rhythm parameters include one or more of the following:

- a parameter indicative of the time variation in the heart rate during a measuring period;
- a parameter indicative of the time variation in the pulse period derived from a PPG signal during a measuring period;
- a parameter indicative of a power spectral density (PSD) of the heart rate variability, in particular a power in a low or very low spectral band;
- a parameter indicative of a pulse transit time (PTT) or indicative of a variability in the pulse transit time, the pulse transit time indicating a length of time between an R peak in an ECG signal, and a reference point between a basal and apex amplitude in a pulse photoplethysmography (PPG) signal following the R peak.

8. The method according to any of claims 1 - 7, wherein the one or more arterial parameters include one or more of the following:

- a parameter relating to a skin temperature at a first location in a body of the subject that is prone to vasoconstriction,
- a parameter indicative of a comparison between a skin temperature at the first location prone to vasoconstriction in the body of the subject and a skin temperature at a second location prone to vasodilation in the body of the subject,
- a parameter indicative of an average pulse transit time (PTT) or indicative of a variation in the pulse transit time, the pulse transit time indicating a length of time between a peak in the R signal in an electrocardiogram, and a mid point between a basal and apex amplitude in a pulse photoplethysmography (PPG) signal following the peak,
- a parameter indicative of a variation in the pulse rising time (PRT), the pulse rising time indicating a length of time between a basal point and an apex point in a pulse photoplethysmography (PPG) signal,
- a parameter indicative of an average pulse wave decreasing time in a PPG signal, the pulse wave decreasing time corresponding to a length of time between a peak and a subsequent basal point in a PPG signal; and
- a parameter indicative of a variation in a pulse width until reflection (PWR), the pulse width until reflection indicating a length of time between a basal point and a dichrotic notch point in a pulse photoplethysmography (PPG) signal.

9. The method according to any of claims 1 - 8, wherein the sweat parameters and the arterial parameters include:

- a parameter indicative of a skin temperature in the first location prone to vasoconstriction,
- a parameter indicative of a pulse transit time (PTT), the pulse transit time indicating a length of time between a peak in the R signal in an electrocardiogram, and a reference point between a basal and apex amplitude in a pulse photoplethysmography (PPG) signal following the peak
- a parameter indicating an average heart rhythm.

10. A computer program for determining a stress indicator in a subject, the computer program comprising instructions which, when the program is executed by a computer, cause the computer to carry out a method comprising:

- receiving from one or more sensors heart rhythm signals indicative of a heart rhythm of the subject;
- receiving from one or more sensors perspiration signals indicative of a level of perspiration of the subject;
- receiving from one or more sensors blood vessel signals indicative of a level of widening or narrowing of blood vessels in the subject;
- determining from the heart rhythm signals one or more heart rhythm parameters indicative of heart rhythm variability,
- determining from the perspiration signals one or more sweat parameters,
- determining from the blood vessel signals one or more arterial parameters, indicative of a level of widening or narrowing of blood vessels in the subject,
- determining a weighted function of the heart rhythm parameters, the sweat parameters and the arterial parameters to define the stress indicator.

11. The computer program according to claim 10, wherein the weighted function indicates a level of stress on a standardized stress scale, optionally the perceived stress scale (PSS), the Visual Analogue Scale for Stress (VASS), or the State-Trait Anxiety Inventory test (STAI), or a combination of standardized stress scales.

12. The computer program according to claim 10 or 11, wherein the weighted function indicates a level of stress on a combined stress scale, the combined stress scale is a weighted sum of standardized stress scales and biochemical markers related to stress.

13. A system for determining a stress indicator in a subject comprising

- one or more heart rhythm sensors for non-invasively measuring a heart rhythm of the subject;
- one or more sweat sensors for non-invasively measuring a level of perspiration of the subject;
- one or more arterial sensors for non-invasively measuring one or more arterial parameters indicating a level of widening or narrowing of blood vessels in the subject; and
- a computing module for

  - receiving from one or more sensors heart rhythm signals indicative of a heart rhythm of the subject;
  - receiving from one or more sensors perspiration signals indicative of a level of perspiration of the subject;
  - receiving from one or more sensors blood vessel signals indicative of a level of widening or narrowing of blood vessels in the subject;
  - determining from the heart rhythm signals one or more heart rhythm parameters indicative of heart rhythm variability,
  - determining from the perspiration signals one or more sweat parameters,
  - determining from the blood vessel signals one or more arterial parameters, indicative of a level of widening or narrowing of blood vessels in the subject,
  - determining a weighted function of the heart rhythm parameters, the sweat parameters and the arterial parameters to define the stress indicator.

14. The system according to claim 13, wherein the sensors comprise a pulse oximeter, an ECG sensor, and one or more thermometers for measuring skin temperature and optionally a skin conductance sensor.

15. The system according to claim 13 or 14, wherein the system is integrated in a wearable device, and optionally comprises a display.

FIG. 1

FIG. 2

FIG. 3

FIG. 4

Europäisches
Patentamt

European
Patent Office

Office européen
des brevets

## EUROPEAN SEARCH REPORT

Application Number

EP 18 38 2132

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/338640 A1 (CHAN ALEXANDER [US] ET AL) 24 November 2016 (2016-11-24) * abstract * * paragraphs [0017], [0019], [0020], [0023], [0025], [0034], [0035], [0055], [0059], [0066], [0067] * ----- | 1-3,5-15 | INV. A61B5/02 A61B5/024 A61B5/16 A61B5/00 |
| X | US 2015/238140 A1 (LABELLE JEFFREY T [US] ET AL) 27 August 2015 (2015-08-27) * paragraphs [0018], [0026], [0027], [0029], [0040], [0041] * ----- | 1,10,13 | ADD. A61B5/01 A61B5/0205 A61B5/021 A61B5/053 |
| X | US 2017/224232 A1 (MAAREK ALBERT [US]) 10 August 2017 (2017-08-10) * paragraphs [0043] - [0048] * ----- | 1,4,10, 13 | |

TECHNICAL FIELDS
SEARCHED     (IPC)

A61B

The present search report has been drawn up for all claims

| Place of search | Date of completion of the search | Examiner |
|---|---|---|
| The Hague | 31 August 2018 | Kowalczyk, Szczepan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 38 2132

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2018

| Patent document cited in search report | | | Publication date | Patent family member(s) | | | Publication date |
|---|---|---|---|---|---|---|---|
| US 2016338640 | A1 | | 24-11-2016 | NONE | | | |
| US 2015238140 | A1 | | 27-08-2015 | US<br>WO | 2015238140<br>2014022586 | A1<br>A1 | 27-08-2015<br>06-02-2014 |
| US 2017224232 | A1 | | 10-08-2017 | NONE | | | |

For more details about this annex : see Official Journal of the European Patent Office, No. 12/82